# EUROPÄISCHE PATENTSCHRIFT

(11) **EP 3 919 001 B1**
(45) Veröffentlichungstag und Bekanntmachung des Hinweises auf die Patenterteilung: **09.08.2023**
(21) Anmeldenummer: 21177274.4
(22) Anmeldetag: 01.06.2021
(51) Int. Cl.: A61B 6/00

(54) **KREISBOGENFÖRMIGES HYBRID-C-PROFIL FÜR EIN C-BOGEN-RÖNTGENGERÄT**
ARC-SHAPED HYBRID C-PROFILE FOR A C-ARM X-RAY DEVICE
PROFILÉ C HYBRIDE CIRCULAIRE POUR UN APPAREIL À RAYONS X À BRAS C

(30) Priorität: 04.06.2020 DE 102020003363
(43) Veröffentlichungstag der Anmeldung: 08.12.2021
(73) Patentinhaber: Ziehm Imaging GmbH, 90471 Nürnberg (DE)
(72) Erfinder: Sander, Thomas, 90453 Nürnberg (DE); Hauser, Ewald, 90451 Nürnberg (DE)
(74) Vertreter: Wittmann, Günther

(56) Entgegenhaltungen:
- EP-B1- 1 372 485
- DE-A1-102016 200 442
- DE-A1-102017 211 764
- DE-U1-202015 008 455

## Beschreibung

### Gebiet der Erfindung

C-Bogen-Röntgengeräte weisen auf einem Fahrgestell eine mehrfach verstellbare Halterung mit einer Lagereinheit für einen kreisbogenförmigen C-Bogen auf, der in der Lagereinheit vorzugsweise motorisch längs seines Umfanges verstellbar gelagert ist und der an seinen gegenüber liegenden Enden einen Röntgenstrahler und einen Röntgendetektor trägt. Soll ein solches C-Bogen-Röntgengerät zur Aufnahme von Projektionsaufnahmen verwendet werden, aus denen ein 3D-Bilddatensatz rekonstruierbar ist, so muss dabei die Aufnahmegeometrie der Projektionsaufnahmen reproduzierbar aus der Kinematik des C-Bogen-Röntgengerätes bestimmbar sein. Bei unterschiedlichen Orientierungen des C-Bogens im Raum soll sich dabei die kreisbogenförmige Gestalt des C-Bogens in einem möglichst geringen Maß durch die auftretenden Drehmomente verändern. Ein Drehmomentvektor in radialer Richtung des C-Bogens führt zu einer Verwindung des C-Profils und damit zu einer Abweichung des C-Profils von der ebenen Kreissegmentform und ein Drehmomentvektor senkrecht zu der Ebene des C-Profils führt zu einer Vergrößerung oder Verkleinerung der Maulweite des C-Bogens. Dabei ist es bei mobilen C-Bogen-Röntgengeräten erforderlich, einen Kompromiss zwischen der Gesamtmasse des C-Bogens und seiner Steifigkeit zu finden, weil bei zunehmender Masse des C-Bogens auch die Massen des Fahrgestells und der Lager- und Verstellelemente des C-Bogen-Röntgengerätes zunehmen, was die Manövrierfähigkeit des mobilen C-Bogen-Röntgengerätes beeinträchtigt.

### Stand der Technik

Aus dem Dokument DE102010021657A1 ist ein C-Bogen-Hohlprofil mit rechteckförmigem Querschnitt bekannt, bei dem schräg zu den Profilflächen gelagerte Laufrollen vorgesehen sind.

Aus dem Dokument DE102017211764A1 ist ein C-Bogen-Profil bekannt, bei dem Verstärkungsprofile aus Stahl in Aluminium eingegossen sind. Die Führungsvorsprünge 8 des Aluminium-Gussprofils, an dem die Laufrollen angreifen, weisen keine Verstärkungen aus Stahl auf.

Aus dem Dokument EP1372485B1 ist ein durch Stahlverstärkungen an den Laufflächen bekanntes Aluminiumprofil bekannt.

Aus dem Dokument DE202015008455U1 ist ein Aluminiumhohlprofil mit einem rechteckförmigen Querschnitt bekannt, bei dem an der Außenseite des Profils außerhalb der Laufflächen der Laufrollen CFK-Platten aufgeklebt sind.

Aus dem Dokument DE102016200442A1 ist ein C-Bogen-Profil aus Aluminium mit eingelegten Stahl-Laufdrähten bekannt.

Aus dem Dokument DE102016200442A1 ist ein C-Bogen-Profil mit rechteckigem Querschnitt aus Aluminium mit eingelegten Stahl-Laufdrähten bekannt, bei dem die Ebenen der Laufrollen schräg zu den Begrenzungsebenen des Rechteckprofils angeordnet sind.

Aus dem Dokument DE9218237U1 ist ein Strangpressprofil mit Laufdrähten aus Stahl für einen C-Bogen bekannt.

Aus dem Dokument DE4214858C1 ist ein Kohlefaser-verstärktes CFK-Profil mit Laufdrähten aus Stahl bekannt.

Aus dem Dokument DE202011002199U1 ist ein Rechteckprofil für einen C-Bogen bekannt, das aus 4 miteinander verschweißten Stahlblechen gebildet wird. Vier weitere Stahlbleche sind an dem Rechteckprofil unter Bildung von Laufflächen für die Laufrollen angeschweißt.

Aus dem Dokument DE29802014U1 der Anmelderin ist ein C-Bogen-Profil mit eingespannten Laufdrähten und einem Hohlprofil aus Aluminium oder faserverstärktem Kunststoff bekannt.

Aus dem Dokument DE102009054360B4 ist eine Nut zur Führung eines Orbitalantriebsriemens in der konvexen Außenfläche des C-Bogens bekannt.

### Beschreibung der Erfindung

Aufgabe der Erfindung ist es, ein kreisbogenförmiges C-Profil für ein C-Bogen-Röntgengerät zu schaffen, das bei gleicher Masse gegenüber bekannten C-Profilen eine höhere Belastbarkeit und Verwindungssteifigkeit aufweist.

Die Aufgabe der Erfindung wird durch ein Hybrid-C-Profil gelöst, bei dem ein die Laufflächen für die Orbital-Laufrollen einer Lagereinheit zur Verfügung stellendes Stahlprofil mit einem Leichtbauprofil durch Verbindungsmittel miteinander verbunden ist.

Die Kraftlinien der von den Laufrollen in das C-Bogen-Profil eingeleiteten Kräfte verlaufen dabei ausschließlich durch ein hoch belastbares Material wie Stahl oder Edelstahl, wodurch die bei den aus dem Stand der Technik bekannten C-Bogen-Profilen mit Kraftlinienverläufen durch Leichtmetall vorhandene Gefahr der Verformung des Leichtmetalls vermieden wird.

Das Stahlprofil wird aus einem zylindermantelförmig gekrümmten Flachprofil mit seitlich angeformten Laufflächen gebildet. Im Rahmen der Erfindung ist vorgesehen, das Stahlprofil derart auszubilden, dass auf der konkaven Seite zwei kreisringförmige Halteprofile abstehen.

Die Aufgabe der Erfindung wird durch eine Vorrichtung mit den im Patentanspruch 1 angegebenen Merkmalen gelöst. Vorteilhafte Ausbildungen werden durch die abhängigen Patentansprüche angegeben Dabei ist vorgesehen, das C-Profil als Hybridbauteil aufzubauen, wobei ein Leichtbauprofil als Grundkörper mit einem als Stahlprofil ausgeführten Tragprofil form-, stoff- und/oder kraftschlüssig miteinander verbunden sind.

Es ist im Rahmen der Erfindung vorgesehen, das Leichtbauprofil als Hohlprofil auszubilden. Es ist weiterhin vorgesehen, als Leichtbauprofil ein auf seiner konvexen Seite zum Stahlprofil hin offenes u- oder v-Profil vorzusehen und ein Hohlprofil durch die Abdeckung des offenen Leichtbauprofils mit dem Stahlprofil zu bilden. Dabei sind zur Aussteifung des offenen u- oder v-Profiles erste Aussteifungen unter Bildung von Kammern vorgesehen.

Es ist vorgesehen, als Leichtbauprofil ein Strangpressprofil aus einer Leichtmetalllegierung, insbesondere einer Aluminium,- oder Magnesiumlegierung, ein Druckguss- oder ein Sandgussprofil aus einer Aluminium,- oder Magnesiumlegierung oder ein Profil aus einem faser-oder partikelverstärkten Kunststoff zu verwenden.

Das Leichtbauprofil weist dabei zur Verbesserung der Belastbarkeit und der Verwindungssteifigkeit innen liegende flächige Aussteifungen auf. Die Querschnittsfläche des Leichtbauprofils kann dabei der Beanspruchung des C-Bogens angepasst werden.

Das Stahlprofil aus einem zylindermantelförmig gekrümmten Flachprofil weist einen rechteckigen Querschnitt unter Bildung von Laufflächen für tragende Zylinderrollen und führende Führungsrollen auf.

Es ist vorgesehen, das Stahlprofil mit einem Querschnitt in der Form des griechischen Buchstabens Pi auszubilden, wobei das Stahlprofil aus einem zylindermantelförmig gekrümmten Flachprofil mit seitlich angeformten Laufflächen und zwei auf der konkaven Seite vorzugsweise orthogonal abstehenden, kreisringförmigen Halteprofilen gebildet wird. Dabei ist vorgesehen, die beiden Halteprofile flächig mit einer Wand der Leichtbauprofils zu verbinden oder die Halteprofile als Federn in korrespondierende Nuten des Leichtbauprofils kraftschlüssig einzufügen und die Nut- und Feder-Verbindung zusätzlich kraftschlüssig, formschlüssig und/oder stoffschlüssig unter Bildung eines Hybrid-C-Profils zu fixieren.

Es ist vorgesehen, das Stahlprofil durch Umformprozesse aus einem Flachmaterial durch Biegen, Walzen, Rollen und/oder Abkanten herzustellen.

Das Flachmaterial kann in den Bereichen der zu bildenden Federn gestanzt und/oder genibbelt und/oder gelasert sein.

Das Stahlprofil wird aus einem homogenen Stahl- oder Edelstahlmaterial gebildet; es ist aber auch vorgesehen, das Stahlprofil aus einem plattierten Material aus Stahl und Edelstahl zu bilden.

Die Verbindung des Leichtbauprofils mit dem Stahlprofil kann stoffschlüssig ausgeführt sein, beispielsweise durch Einbringen eines Klebstoffes in die Nut- und Federverbindungen oder durch Verkleben korrespondierender Flächen des Leichtbauprofils und des Stahlprofils. Dabei kann zur Verhinderung des Abschälens des ausgehärteten Klebstoffes vorgesehen sein kann, die Nut oder/und die Feder oder/und die korrespondierenden Flächen von Leichtbauprofil und Stahlprofil hinterschnittartig zu profilieren, beispielsweise zu rillen oder die Feder oder wenigstens eine korrespondierende Fläche zu lochen oder zu schlitzen. Es ist weiterhin vorgesehen, dass die Verbindung stoffschlüssig durch Löten erfolgt. Weiterhin ist vorgesehen, das Leichtbauprofil mit dem Stahlprofil durch Nieten, Reibschweisssnieten, Nageln, Schrauben und/oder Verstiften formschlüssig zu verbinden. Weiterhin ist vorgesehen, das Stahlprofil und das Leichtbauprofil durch Pressen im Bereich der Nut im Leichtbauprofil kraftschlüssig zu verbinden. Es ist darüber hinaus eine kombinierte Verwendung von verschiedenen Verbindungsarten vorgesehen.

Bei der Herstellung des C-Profils ist vorgesehen, mittels einer Haltevorrichtung für das Leichtbauprofil das Stahlprofil unter elastischer Verformung auf des Leichtbauprofil aufzupressen und das Stahlprofil auf dem Leichtbauprofil nach Prüfung der Maßhaltigkeit des Hybrid-C-Profils, insbesondere der Maßhaltigkeit bezüglich der zylindermantelförmigen Laufflächen zu fixieren.

### Kurze Beschreibung der Figuren

Die Erfindung wird nun unter Bezugnahme auf die beigefügten Figuren detaillierter beschrieben, die nicht beschränkende Ausführungsformen zeigen, wobei:
Fig. 1 ein erfindungsgemäßes Hybrid-C-Profil mit Tragrollen, zeigt;
Fig. 2 eine perspektivische Darstellung einer weiteren Ausführungsform des erfindungsgemäßen Hybrid-C-Profils mit einem Kühlmittelkanal, ist;
Fig. 3 ein Ausführungsbeispiel eines Hybrid-C-Profils einem Stahlprofil mit rechteckigem Querschnitt, zeigt;
Fig. 4 ein Hybrid-C-Profil mit einem v-förmigen Leichtbauprofil und einer Nut- und Feder-Verbindung, zeigt;
Fig. 5 ein Hybrid-C-Profil mit einem v-förmigen Leichtbauprofil und kreisringförmigen Verbindungsstellen, zeigt;
Fig. 6 Ausführungsbeispiele für die Formgebung der Federn, zeigt; und
Fig. 7 ein Mobiles C-Bogen-Röntgengerät zeigt.

### Detaillierte Beschreibung der Erfindung

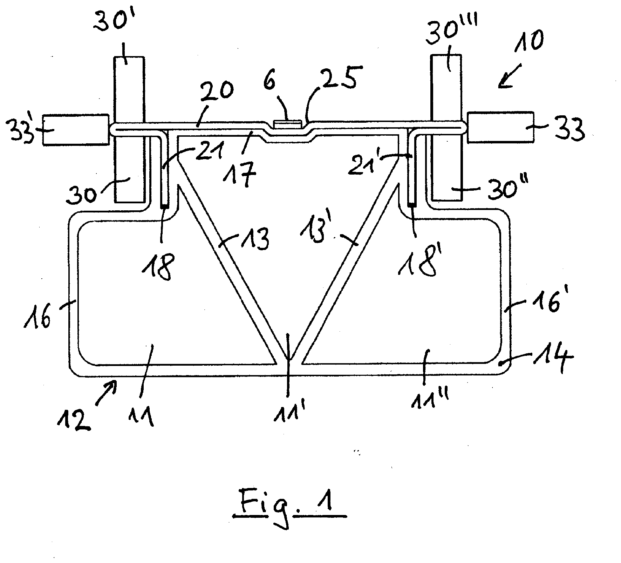

In Fig. 1 ist ein Ausführungsbeispiel eines erfindungsgemäßen Hybrid-C-Profils 10 mit Tragrollen schematisch dargestellt. Das Hybrid-C-Profil 10 wird aus einem Leichtbauprofil 12 und einem Stahlprofil 20 mittels einer unlösbaren Verbindung gebildet. Das Stahlprofil 20, das vorzugsweise aus einem mehrfach gekanteten und gebogenen und gerollten Flachmaterial besteht, weist eine erste Lauffläche 31, 31' und eine gegenüberliegende Lauffläche 32, 32' auf, wobei in einer nicht dargestellten Lagereinheit Zylinderrollen 30, 30', 30", 30"' gelagert sind, die auf den ersten und zweiten Laufflächen 31, 31', 32, 32' spielfrei abrollen. Zur Sicherstellung der Spielfreiheit sind die Achsen der gegenüber liegenden Zylinderrollen 30, 30' und 30", 30" derart in der Lagereinheit gelagert, dass die Zylinderrollen 30, 30', 30", 30" das Stahlprofil 20 mit einem hinreichend großen Anpressdruck halten. Zur seitlichen Führung des Hybrid-C-Profils 10 greifen an einer ersten Seitenkante und an der dazu parallel verlaufenden zweiten Seitenkante des Stahlprofils 20 Führungsrollen 33, 33' an, deren Achsen senkrecht auf den ersten und zweiten Laufflächen 31, 31', 32, 32' stehen. Vorzugsweise sind diese Führungsrollen 33, 33' als Profilrollen ausgeführt. Das Stahlprofil 20 weist in der Mitte des konvexen, zylindrischen Bereiches zwischen den ersten Laufflächen 31, 31' eine Zahnriemen-Führungsnut 25 auf, in der ein Zahnriemen 6 im Bereich außerhalb eines nicht dargestellten Omega-Antriebes versenkt geführt wird.

Das Stahlprofil 20 weist zwischen den zweiten Laufflächen 32, 32' zwei orthogonal zu den ersten und zweiten Laufflächen 31, 31', 32, 32' abstehende, kreisringförmige Halteprofile 22, 22' auf, die bei der Verbindung des Stahlprofils 20 mit dem Leichtbauprofil 12 als Federn 21, 21' in integral an dem Leichtbauprofil 12 angeformte Nuten 18, 18' eingreifen, wobei die so entstandenen Nut- und-Feder-Verbindungen durch zusätzliche Verbindungsmittel, beispielsweise durch eine Klebe-, Niet-, Löt- oder Schweißverbindung unlösbar miteinander verbunden werden.

Als Leichtbauprofil 12 im Ausführungsbeispiel der Fig. 1 ist ein geschlossenes Hohlprofil mit durch erste Aussteifungen 13, 13' und durch eine zweite Aussteifung 17 gebildeten Kanälen 11, 11', 11" vorgesehen. Die Verbindung des Leichtbauprofils 12 mit dem Stahlprofil 20 erfolgt zusätzlich zu der Nut- und Feder-Verbindung durch eine flächige Klebeverbindung im Bereich der zweiten Aussteifung.

Die Kanäle 11, 11' und 11" sind für die Verlegung von Energieversorgungs- und/oder Daten- und/oder Kühlmittelleitungen vorgesehen, wobei die Außenkontur des Leichtbauprofils 12 mit den Seitenwänden 16, 16' als Wärmetauscherfläche vorgesehen ist.

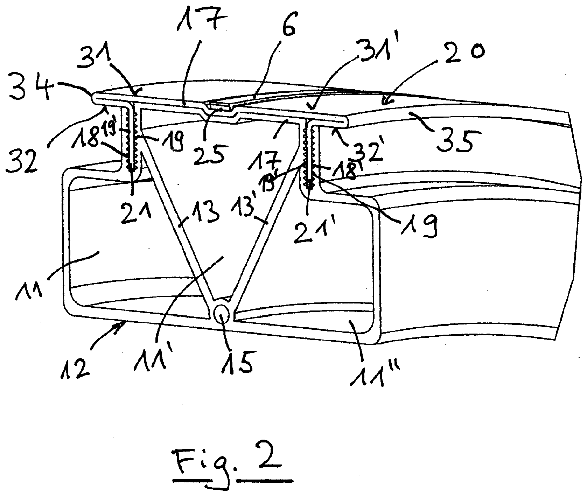

In Fig. 2 ist eine weitere Ausführungsform des erfindungsgemäßen Hybrid-C-Profils 10 im Anschnitt perspektivisch dargestellt. Das Leichtbauprofil 12 weist gegenüber dem Leichtbauprofil 12 aus Fig. 1 einen zusätzlichen Kühlmittelkanal 15 auf, der im Falle der Verwendung von duktilen Leichtmetall-Legierungen für das Leichtbauprofil 12 einen guten Wärmekontakt zu der äußeren Oberfläche des Leichtbauprofils 12 aufweist. Es ist im Rahmen der Erfindung vorgesehen, die Lage und Anzahl der Kanäle zu variieren. Im Bereich der Nuten 18, 18' und der Federn 21, 21' der Nut-und-Feder-Verbindungen sind die Hinterschnitte 19, 19' zur Verbesserung der Kleberhaftung schematisch dargestellt. Weisen sowohl die Nuten 18, 18' als auch die Federn 21, 21' Hinterschnitte auf, so wird dadurch eine Klebefuge mit einem wellenförmigen Querschnitt ausgebildet.

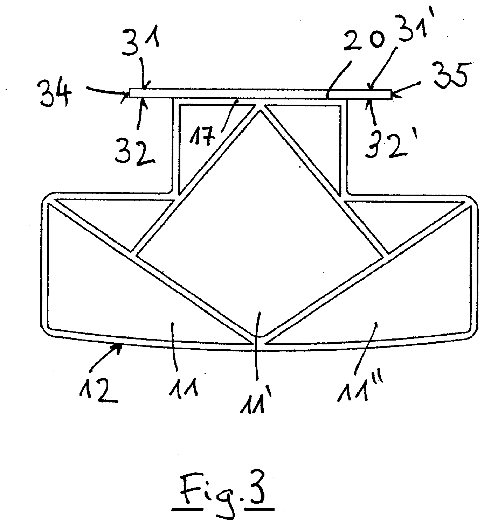

In Fig. 3 ist ein Ausführungsbeispiel für die Verbindung des Stahlprofils 20 mit dem Leichtbauprofil 12 im Bereich der zweiten Aussteifung 17 schematisch dargestellt. Das zylindermantelförmig gebogene Stahlprofil 12 mit rechteckigem Querschnitt liegt an seiner konkaven Zylindermantelfläche an der konvexen Seite des Leichtbauprofils 12 an und ist mittels nicht dargestellter Verbindungsmittel mit diesem verbunden.

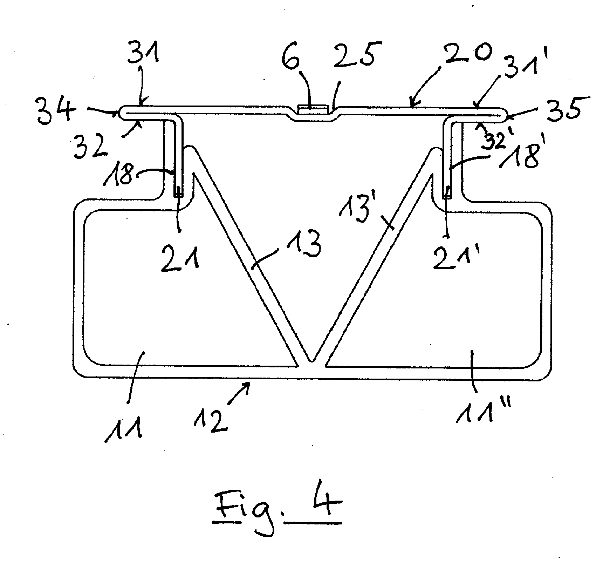

Im Ausführungsbeispiel der Fig. 4 ist als Leichtbauprofil 12 ein durch Weglassen einer zweiten Aussteifung gebildetes offenes Profil in der Form eines u-Profils oder eines v-Profils mit durch erste Aussteifungen 13, 13' gebildeten Kanälen 11, 11" gezeigt. Durch die in Fig. 4 gezeigten Nut-und-Feder-Verbindungen 18, 21 und 18', 21' des Leichtbauprofils 12 mit dem Stahlprofil 20 wird das offene Profil durch das Stahlprofil (20) abgedeckt, wobei ein geschlossenes Hohlprofil und unter Ausbildung eines von dem Leichtbauprofil 12 und dem Stahlprofil 20 begrenzten Kanals gebildet wird.

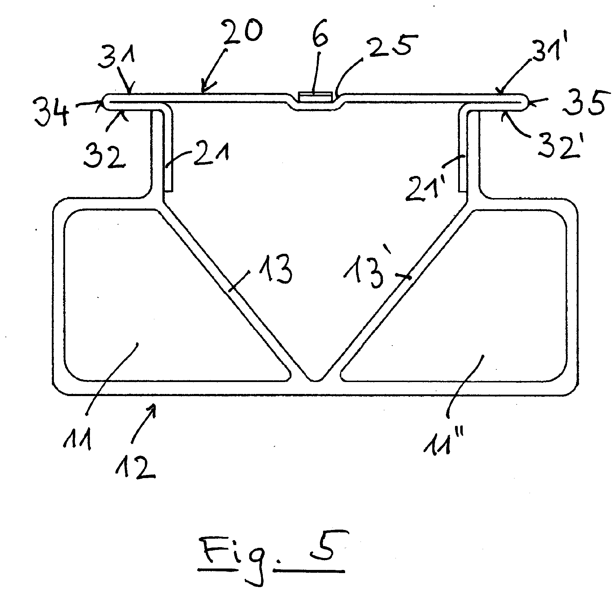

Im Ausführungsbeispiel der Fig. 5 ist als Leichtbauprofil 12 ein durch Weglassen einer zweiten Aussteifung gebildetes offenes u- oder v-Profil mit durch erste Aussteifungen 13, 13' gebildeten Kanälen 11, 11" gezeigt. Das Leichtbauprofil 12 ist mit dem Stahlprofil 20 im Bereich der kreisringförmigen Federn 21, 21 mit kreisringförmigen Flächen des Leichtbauprofils mittels nicht dargestellter Verbindungsmittel verbunden.

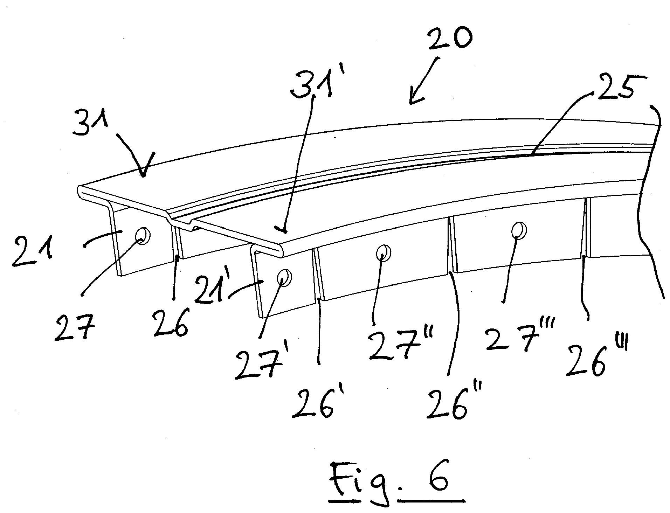

In Fig. 6 ist ein Aufführungsbeispiel für ein erfindungsgemäßes Stahlprofil 20 schematisch dargestellt. Bei den von den Laufflächen 31, 31' vorzugsweise orthogonal abstehenden Federn 21, 21' sind zur Verbesserung der Formgebung des Stahlprofils 20 orthogonal zu den ersten Laufflächen 31, 31' angeordnete Schlitze 26, 26', 26", 26‴ vorgesehen. Die Schlitze können v-förmig ausgebildet sein mit einer breiteren Öffnung auf den den Laufflächen 31, 31' abgewandten Seiten der Federn 21, 21'. Es ist weiterhin vorgesehen, an den Federn 21, 21' Ausnehmungen 27, 27', 27", 27"' für eine bessere Kleberhaftung und/oder zur Erzeugung von Formschlüssen nach der Montage des Stahlprofils 20 einzubringen.

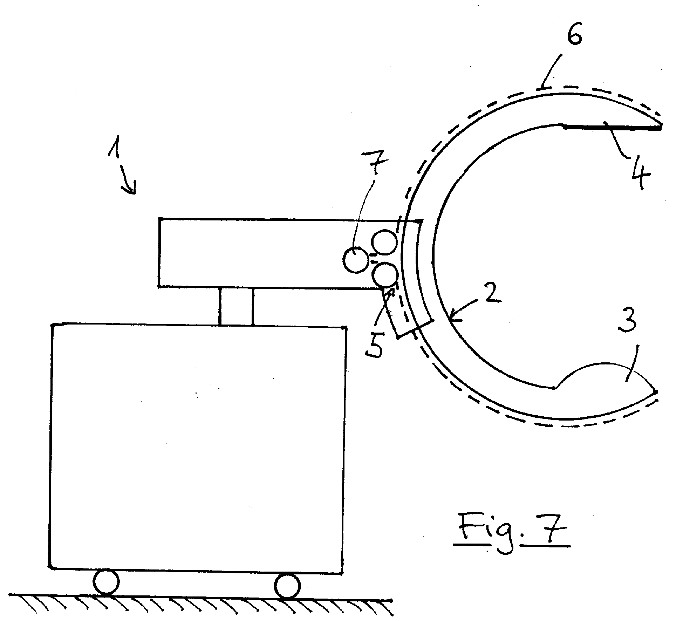

In Fig. 7 ist ein mobiles C-Bogen-Röntgengerät 1 mit einem C-Bogen 2 schematisch dargestellt. An einer mehrfach verstellbaren Lagereinheit 5 ist der C-Bogen 2 längs seines Umfanges verschieblich gelagert. Der C-Bogen 2 trägt an seinem einen Ende einen Röntgenstrahler 3 und an dem gegenüberliegenden Ende einen Röntgenflachdetektor (Flat Panel Detector, FPD) 4. An der konvexen Seite des C-Bogens 2 ist ein Zahnriemen 6 schematisch dargestellt, der an der konvexen Seite des C-Bogens 2 von einem Ende des C-Bogens 2 zu seinem anderen Ende verläuft und an den Enden des C-Bogens fixiert ist. Im Inneren der Lagereinheit 5 wird der Zahnriemen 5 mittels eines Omega-Antriebes 7 gespannt und angetrieben. Der Omega-Antrieb weist einen Motor mit einem Drehgeber auf, mittels dem der C-Bogen 2 reproduzierbar motorisch verstellbar ist.

### Bezugszeichenliste

- 1: C-Bogen-Röntgengerät
- 2: C-Bogen
- 3: Röntgenstrahler
- 4: Röntgenflachdetektor (Flat Panel Detector FPD)
- 5: Lagereinheit
- 6: Zahnriemen
- 7: Omega-Antrieb
- 10: Hybrid-C-Profil
- 11, 11', 11": Kanal
- 12: Leichtbauprofil
- 13, 13': erste Aussteifung
- 14: Querschnittsfläche
- 15: Kühlmittelkanal
- 16, 16': Seitenwand
- 17: zweite Aussteifung
- 18, 18': Nut
- 19, 19': Hinterschnitt
- 20: Stahlprofil
- 21, 21': Feder
- 22, 22': Halteprofil
- 25: Zahnriemen-Führungsnut
- 26, 26', 26", 26"', 26"": Schlitz
- 27, 27', 27", 27"', 27"": Ausnehmung
- 30, 30', 30", 30"': Zylinderrolle
- 31, 31': erste Lauffläche
- 32, 32': zweite Lauffläche
- 33, 33': Führungsrolle
- 34: erste Seitenkante
- 35: zweite Seitenkante

## Patentansprüche

1. Hybrid-C-Profil für einen C-Bogen (2) eines C-Bogen-Röntgengerätes (1), bei dem der C-Bogen (2) an einem Tragprofil in einer Lagereinheit (5) längs seines Umfanges verschieblich geführt wird, aufweisend
- ein kreisbogenförmiges Leichtbauprofil (12) als Grundkörper, und
- ein kreisbogenförmiges Stahlprofil (20) als Tragprofil mit einem zylindermantelförmigen Abschnitt, an dessen konvexer Seite erste Laufflächen (31, 31') und an dessen konkaver Seite zweite Laufflächen (32, 32') für Tragrollen und dessen erste Seitenkante (34) und zweite Seitenkante (35) als Führungsschienen für Führungsrollen (33, 33') der Lagereinheit (5) ausgebildet sind,
wobei das Leichtbauprofil (12) und das Stahlprofil (20) durch Verbindungsmittel miteinander verbunden sind.

2. Hybrid-C-Profil nach Anspruch 1,
**dadurch gekennzeichnet, dass** das Leichtbauprofil (20) zwei kreisbogenförmige Nuten (18, 18') und das Stahlprofil (20) zwei von dem Stahlprofil (20) abstehende Federn (21, 21') aufweist und wobei die Verbindungsmittel als Nut- und Feder-Verbindungen zwischen den Nuten (18, 18') und den Federn (21, 21') ausgeführt sind.

3. Hybrid-C-Profil nach Anspruch 2,
**dadurch gekennzeichnet, dass** die Federn (21, 21') orthogonal zu den ersten Laufflächen (31, 31') und den zweiten Laufflächen (32, 32') verlaufen.

4. Hybrid-C-Profil nach einem der Ansprüche 2 bis 3,
**dadurch gekennzeichnet, dass** die Nuten (18, 18') und die Federn (21, 21') an den zueinander gewandten Flächen Rillen und/oder Profilierungen aufweisen.

5. Hybrid-C-Profil nach einem der Ansprüche 1-4,
**dadurch gekennzeichnet, dass** das Leichtbauprofil (12) ein Hohlprofil ist und im Inneren des Leichtbauprofils (12) erste Aussteifungen (13, 13') und eine zweite Aussteifung (17) unter Bildung von Kanälen (11, 11', 11") zur Aufnahme von Energieversorgungs-, Daten- und Kühlmittelleitungen vorgesehen sind.

6. Hybrid-C-Profil nach einem der Ansprüche 1-5,
**dadurch gekennzeichnet, dass** das Leichtbauprofil (12) ein auf seiner konvexen Seite offenes Profil mit ersten Aussteifungen (13, 13') ist und das offene Profil durch das Stahlprofil (20) unter Bildung eines geschlossenen Hohlprofils abgedeckt wird.

7. Hybrid-C-Profil nach einem der Ansprüche 1-6,
**dadurch gekennzeichnet, dass** das Leichtbauprofil (20) aus einer Leichtmetall-Legierung besteht.

8. Hybrid-C-Profil nach Anspruch 7,
**dadurch gekennzeichnet, dass** die Leichtmetall-Legierung eine Aluminiumlegierung oder eine Magnesiumlegierung ist.

9. Hybrid-C-Profil nach einem der Ansprüche 1-8,
**dadurch gekennzeichnet, dass** das Leichtbauprofil (12) ein mittels Strangpressen hergestelltes Bauteil ist.

10. Hybrid-C-Profil nach einem der Ansprüche 1-6,
**dadurch gekennzeichnet, dass** das Leichtbauprofil (12) ein faser-und/oder partikelverstärktes Kunststoffverbundbauteil ist.

11. Hybrid-C-Profil nach einem der Ansprüche 1-10,
**dadurch gekennzeichnet, dass** das Stahlprofil (20) ein aus einem Flachmaterial durch Biegen, Abkanten, Walzprofilieren, Stanzen und/oder Laserschneiden hergestelltes Profil ist.

12. Hybrid-C-Profil nach einem der Ansprüche 1-11,
**dadurch gekennzeichnet, dass** das Stahlprofil (20) in der Mitte seiner konvexen Oberfläche zwischen den ersten Laufflächen (31, 31') eine Zahnriemen-Führungsnut (25) zur Führung eines Zahnriemens (6) aufweist.

13. Hybrid-C-Profil nach einem der Ansprüche 1-12,
**dadurch gekennzeichnet, dass** die Verbindungsmittel durch Kleben, Löten oder Schweißen hergestellte stoffschlüssige Verbindungen zwischen dem Leichtbauprofil (12) und dem Stahlprofil (20) sind.

14. Hybrid-C-Profil nach einem der Ansprüche 1-13,
**dadurch gekennzeichnet, dass** die Verbindungsmittel durch Zusammenpressen der Nuten (18, 18') hergestellte kraftschlüssige Verbindungen zwischen dem Leichtbauprofil (12) und dem Stahlprofil (20) sind.

15. Hybrid-C-Profil nach einem der Ansprüche 1-14,
**dadurch gekennzeichnet, dass** die Verbindungsmittel mittels Nieten, Schrauben, Nageln, Verstiften hergestellte formschlüssige Verbindungen zwischen dem Leichtbauprofil (12) und dem Stahlprofil (20) sind.

## Claims

1. A hybrid C-profile for a C-arm (2) of a C-arm X-ray apparatus (1), in which the C-arm (2) is guided displaceably along its circumference on a support profile in a bearing unit (5), comprising
- a circular-arc-shaped lightweight profile (12) as a basic body, and
- a circular-arc-shaped steel profile (20) as a supporting profile with a cylinder-shell-shaped section, on the convex side of which first running surfaces (31, 31') and on the concave side of which second running surfaces (32, 32') for supporting rollers are formed, and the first side edge (34) and second side edge (35) of which are formed as guide rails for guide rollers (33, 33') of the bearing unit (5),
wherein the lightweight profile (12) and the steel profile (20) are connected to each other by connecting means.

2. The hybrid C-profile according to claim 1,
**characterized in that** the lightweight profile (20) comprises two circular arc-shaped grooves (18, 18') and the steel profile (20) comprises two tongues (21, 21') projecting from the steel profile (20), and wherein the connecting means are designed as tongue-and-groove connections between the grooves (18, 18') and the tongues (21, 21').

3. The hybrid C-profile according to claim 2,
**characterized in that** the tongues (21, 21') run orthogonally to the first running surfaces (31, 31') and the second running surfaces (32, 32').

4. The hybrid C-profile according to one of claims 2 to 3,
**characterized in that** the grooves (18, 18') and the tongues (21, 21') comprise gouges and/or profiling on the surfaces facing one another.

5. The hybrid C-profile according to one of claims 1 to 4,
**characterized in that** the lightweight profile (12) is a hollow profile and first stiffeners (13, 13') and a second stiffener (17) are provided in the interior of the lightweight profile (12) to form channels (11, 11', 11") for accommodating power supply lines, data lines and coolant lines.

6. The hybrid C-profile according to one of claims 1 to 5,
**characterized in that** the lightweight profile (12) is a profile open on its convex side with first stiffeners (13, 13') and the open profile is covered by the steel profile (20) to form a closed hollow profile.

7. The hybrid C-profile according to one of claims 1 to 6,
**characterized in that** the lightweight profile (20) consists of a light metal alloy.

8. The hybrid C-profile according to claim 7,
**characterized in that** the light metal alloy is an aluminum alloy or a magnesium alloy.

9. The hybrid C-profile according to any one of claims 1 to 8,
**characterized in that** the lightweight profile (12) is a component produced by extrusion.

10. The hybrid C-profile according to one of the claims 1 to 6,
**characterized in that** the lightweight profile (12) is a fiber- and/or particle-strengthened plastic composite component.

11. The hybrid C-profile according to one of the claims 1 to 10,
**characterized in that** the steel profile (20) is a profile made from a flat material by bending, bending, roll forming, punching and/or laser cutting.

12. The hybrid C-profile according to any one of claims 1 to 11,
**characterized in that** the steel profile (20) comprises a toothed belt guide groove (25) for guiding a toothed belt (6) in the center of its convex surface between the first running surfaces (31, 31').

13. The hybrid C-profile according to one of the claims 1 to 12,
**characterized in that** the connecting means are positive substance jointings between the lightweight profile (12) and the steel profile (20) produced by bonding, soldering or welding.

14. The hybrid C-profile according to one of the claims 1 to 13,
**characterized in that** the connecting means are friction-lock connections between the lightweight profile (12) and the steel profile (20) produced by pressing the grooves (18, 18') together.

15. The hybrid C-profile according to one of the claims 1 to 14,
**characterized in that** the connecting means are form-fit connections between the lightweight profile (12) and the steel profile (20) produced by means of rivets, screws, nails, pins.

## Revendications

1. Profil en C hybride pour un bras en C (2) d'un appareil à rayons X à bras en C (1), dans lequel le bras en C (2) est guidé de manière à pouvoir être déplacé le long de sa circonférence sur un profil de support dans une unité porteuse (5), comprenant :
- un profil de poids léger en forme d'arc circulaire (12) en tant que corps de base ; et
- un profil en acier en forme d'arc circulaire (20) en tant que profil de support présentant une section en forme d'enveloppe cylindrique, sur le côté convexe duquel des premières surfaces de glissement (31, 31') et sur le côté concave duquel des secondes surfaces de glissement (32, 32') pour supporter des rouleaux sont formées, et dont le premier bord latéral (34) et le second bord latéral (35) sont formés en tant que rails de guidage pour des rouleaux de guidage (33, 33') de l'unité porteuse (5) ;
dans lequel le profil de poids léger (12) et le profil en acier (20) sont connectés l'un à l'autre par des moyens de connexion.

2. Profil en C hybride selon la revendication 1,
**caractérisé en ce que** le profil de poids léger (20) comprend deux gorges en forme d'arc circulaire (18, 18') et le profil en acier (20) comprend deux languettes (21, 21') qui font saillie depuis le profil en acier (20), et dans lequel les moyens de connexion sont conçus en tant que connexions languette-et-gorge entre les gorges (18, 18') et les languettes (21, 21').

3. Profil en C hybride selon la revendication 2,
**caractérisé en ce que** les languettes (21, 21') courent orthogonalement aux premières surfaces de glissement (31, 31') et aux secondes surfaces de glissement (32, 32').

4. Profil en C hybride selon l'une quelconque des revendications 2 et 3,
**caractérisé en ce que** les gorges (18, 18') et les languettes (21, 21') comprennent des rainures et/ou un profilage sur les surfaces se faisant mutuellement face.

5. Profil en C hybride selon l'une quelconque des revendications 1 à 4,
**caractérisé en ce que** le profil de poids léger (12) est un profil creux et des premiers éléments de renforcement (13, 13') et un second élément de renforcement (17) sont prévus sur l'intérieur du profil de poids léger (12) pour former des canaux (11, 11', 11") pour loger des lignes d'alimentation électrique, des lignes de données et des lignes de liquide de refroidissement.

6. Profil en C hybride selon l'une quelconque des revendications 1 à 5,
**caractérisé en ce que** le profil de poids léger (12) est un profil ouvert sur son côté convexe à l'aide de premiers éléments de renforcement (13, 13') et le profil ouvert est recouvert par le profil en acier (20) pour former un profil creux fermé.

7. Profil en C hybride selon l'une quelconque des revendications 1 à 6,
**caractérisé en ce que** le profil de poids léger (20) est constitué en un alliage en métal léger.

8. Profil en C hybride selon la revendication 7,
**caractérisé en ce que** l'alliage en métal léger est un alliage d'aluminium ou un alliage de magnésium.

9. Profil en C hybride selon l'une quelconque des revendications 1 à 8,
**caractérisé en ce que** le profil de poids léger (12) est un composant produit par extrusion.

10. Profil en C hybride selon l'une quelconque des revendications 1 à 6,
**caractérisé en ce que** le profil de poids léger (12) est un composant composite en matière plastique renforcée par fibres et/ou particules.

11. Profil en C hybride selon l'une quelconque des revendications 1 à 10,
**caractérisé en ce que** le profil en acier (20) est un profil réalisé à partir d'un matériau plat par cintrage, laminage, poinçonnage et/ou découpe au laser.

12. Profil en C hybride selon l'une quelconque des revendications 1 à 11,
**caractérisé en ce que** le profil en acier (20) comprend une gorge de guidage de courroie dentée (25) pour guider une courroie dentée (6) au centre de sa surface convexe entre les premières surfaces de glissement (31, 31').

13. Profil en C hybride selon l'une quelconque des revendications 1 à 12,
**caractérisé en ce que** les moyens de connexion sont des jointures en substance positive entre le profil de poids léger (12) et le profil en acier (20) qui sont produites par cintrage, brasage ou soudage.

14. Profil en C hybride selon l'une quelconque des revendications 1 à 13,
**caractérisé en ce que** les moyens de connexion sont des connexions à verrouillage par friction entre le profil de poids léger (12) et le profil en acier (20) qui sont produites en pressant les rainures (18, 18') ensemble.

15. Profil en C hybride selon l'une quelconque des revendications 1 à 14,
**caractérisé en ce que** les moyens de connexion sont des connexions à emboîtement par complémentarité de formes entre le profil de poids léger (12) et le profil en acier (20) qui sont produites au moyen de rivets, de vis, de clous, de pointes.
